# EUROPEAN PATENT SPECIFICATION

(11) **EP 1 230 855 B1**
(45) Date of publication and mention of the grant of the patent: **01.10.2008**
(21) Application number: 00976305.3
(22) Date of filing: 16.11.2000
(51) Int. Cl.: A01N 25/04, A01N 25/10, A01N 25/22, A61K 9/10, C08F 20/12, C08L 33/04, A01N 35/02, A01N 37/02, A01N 43/20, A61K 47/32, C08F 220/18

(54) **SUSTAINED-RELEASE PREPARATION OF AQUEOUS DISPERSION TYPE AND PROCESS FOR PRODUCING THE SAME**
ZUBEREITUNG VOM WÄSSRIGEN DISPERSIONSTYP MIT VERZÖGERTER FREIGABE UND VERFAHREN ZU IHRER HERSTELLUNG
PREPARATION DU TYPE DISPERSION AQUEUSE A LIBERATION PROLONGEE, ET PROCEDE DE PRODUCTION CORRESPONDANT

(30) Priority: 19.11.1999 JP 32982799; 16.06.2000 JP 2000182127
(43) Date of publication of application: 14.08.2002
(73) Proprietor: NOF CORPORATION, Tokyo 150-0013 (JP); Shinetsu Chemical Co., Ltd., Tokyo 100-0004 (JP)
(72) Inventor: TAMURA, Toru, Chita-gun, Aichi 470-2345 (JP); TASAKA, Tomohisa, Handa-shi, Aichi 475-0912 (JP); NAKAMURA, Tetsuya, Handa-shi, Aichi 475-0962 (JP); SUGIURA, Motoyuki, Minowa-cho, Anjo-shi, Aichi 446-0051 (JP)
(74) Representative: Müller-Boré & Partner Patentanwälte
(86) International application number: PCT/JP2000/008102
(87) International publication number: WO 2001/037660

(56) References cited:
- EP-A2- 0 046 136
- EP-A2- 0 347 145
- EP-A2- 0 816 430
- WO-A1-95/30411
- WO-A1-98/00170
- JP-A- 1 315 402
- JP-A- 8 038 865
- JP-A- 61 181 812
- JP-A- 62 072 710
- US-A- 4 575 539
- DATABASE WPI Section Ch, Week 199120 Derwent Publications Ltd., London, GB; Class A14, AN 1991-143954 XP002306357 & JP 03 079608 A (SHINGIJUTSU KAIHATSU KK) 4 April 1991 (1991-04-04)
- YAUNG J.F. ET AL.: 'pH-sensitive hydrogels based on polyvinylpyrrolidone-polyacrylic acid(PVP-PAA) semi-interpenetrating networks)Semi-IPN): Swelling and controlled release' JOURNAL OF APPLIED POLYMER SCIENCE vol. 69, no. 5, 1998, pages 921 - 930, XP002936549
- MASAHIKO KAWASHIMA ET AL.: 'Boujun no ondo izonsei seigyo wo mokuteki to shita suiso ketsugousei koubunshi IPN no bunshi sekkei' KOUBUNSHI GAKKAI YOKOUSHUU vol. 41, no. 3, 1995, page 763, XP002936550

## Description

This invention relates to an aqueous dispersion-type sustained release preparation which can release almost all the functional substance contained inside of its interpenetrating polymer networks (hereinbelow referred to as "IPN") at a constant speed for a considerable period of time, and easily applicable by spraying or scattering, as well as be able to be given more functional properties such as adhesivity etc. This invention also relates to methods for producing them.

In recent years, the development of a sustained release preparation containing a functional substance such as pheromones, medicines, agricultural chemicals and fragrant materials etc. has been desired. By now, lots of methods have been proposed, for example, in which functional substances are micro-encapsulated with cellulose derivatives to produce sustained release preparations (JP 58183601), or in which functional substances-impregnatedpellets of synthetic resin compatible with the functional substances are crushed into pieces before being covered their surfaces with inorganic powders or synthetic resin incompatible with the functional substances (JP 6192024), or in which pellets of synthetic resin containing functional substances are mixed and suspended with O/W-type acrylic adhesive emulsion (JP 7231743).

However, the micro-encapsulated sustained release preparations disclosed in JP 58 183601 are unsatisfactory as sustained release preparations because of the poor controllability of the release speed of the functional substances and the lower mechanical strength of the capsules covering their surfaces.

The sustained release preparations disclosed in JP 6192024 are prevented from blocking, but they have a problem that secondary elaboration to cover their surface becomes necessary which increases the steps for producing them.

On the other hand, the aqueous dispersibility of the sustained release preparations disclosed in JP 7 231 743 are so poor that they easily become spotty when being applied by spraying or scattering, and that also needs a step to miking them. EP 0 347 145 describes sustained release preparations, comprising an interpenetrating polymer gel formed from a hydrophilic component and a hydrophobic component and a functional substance contained therein. EP 0 046 136 describes interpenetrating polymer blend compositions, comprising a water swellable polymer substrate and a less permeable condensation polymers for the use as drug releasing agents. WO 95/30411 describes a transdermal drug delivery system, comprising a polyelectrolyte gel matrix, composed of two different polymer components which form an interpenetrating polymer network, and the loading of an ionizable drug thereon. WO 98/00170 describes a biodegradable hydrogel, composed of two different interpenetrating polymer networks interconnected to one another through hydrolysable spacers. US 4 575 539 describes a drug delivery system, comprising an interpenetrating polymer network, provided through swelling of hydrogel beads with an acrylic swelling agent and successive cross-linking of said swelling agent using a cross-linking agent. JP 3079608 describes a preparation of an acrylamide/acrylic acid interpenetrating network polymer useful as a drug delivery system, wherein acrylamide, alkyl methacrylate or alkyl acrylate are dissolved in a solvent together with a cross-linking agent and a polymerization initiator.

The purpose of this invention is to provide an aqueous dispersion-type sustained release preparation which can release almost all the functional substance contained inside of its interpenetrating polymer networks (hereinbelow referred to as "IPN") at a constant speed for a considerable period of time, and easily applicable by spraying or scattering, as well as be able to be given more functional properties such as adhesivity etc. This invention also aims at providing a method for producing the same.

### SUMMARY OF THE INVENTION

To solve the problems described above, the first aspect of this invention is an aqueous dispersion-type sustained release preparation as defined in claim 1 which contains a functional substance inside of its interpenetrating polymer networks composed of microgel A and microgel B, wherein said microgel A is formed by monomer components which have good affinity to said functional substance whereas saidmicrogel B is formed by monomer components which have poor affinity to said functional substance, wherein said microgel A and microgel B in the interpenetrating polymer networks are chemically bonded with each other through a fragment of (meth) acrylic ester monomers having a photo-polymerizing group (component a1) or radical-polymerizing organic peroxides (component a2).

Said microgel A is formed by monomer components which comprise the one selected from (meth) acrylic ester monomers having a photo-initiating group (component a1) and radical-polymerizing organic peroxides (component a2), and also comprise a (meth)acrylic ester monomer (component b), as well as a polyfunctional (meth) acrylic ester monomer (component c). Optionally, said microgel A is formed by monomer components which further comprise a hydrophilic monomer (component d). Another aspect of this invention is an aqueous dispersion-type sustained release preparation wherein said microgel B is formed by monomer components which comprise a (meth)acrylic ester monomer (component e) and a polyfunctional (meth)acrylic ester monomer (component f).

A further aspect of this invention is an aqueous dispersion-type sustained release preparation wherein said functional substance is a pheromone, and said (meth)acrylic ester monomer (component b) of said microgel A has an alkyl chain having 6-20 carbon atoms.

A still further aspect of this invention is an aqueous dispersion-type sustained release preparation wherein said functional substance is a pheromone, and said (meth)acrylic ester monomer (component e) of said microgel B has an alkyl chain having 1-8 carbon atoms.

Another aspect of this invention is an aqueous dispersion-type sustained release preparation wherein the pH value of the emulsion of said aqueous dispersion-type sustained release preparation is 2-9.

The second aspect of this invention is a method for producing an aqueous dispersion-type sustained release preparation containing a functional substance inside of its interpenetrating polymer networks comprising: A first step of emulsion copolymerization using monomer components which comprise the one selected from (meth)acrylic ester monomers having a photo-initiating group (component a1) and radical-polymerizing organic peroxides (component a2), and also comprise a (meth)acrylic ester monomer (component b), a polyfunctional (meth)acrylic ester monomer (component c), and optionally, a hydrophilic monomer (component d), as well as a functional substance, a surface active agent, a polymerization initiator and water, to obtain an emulsion;
A second step of polymerization by irradiation of activated energy lines to the emulsion obtained in the first step after being added with a (meth)acrylic ester monomer (component e) and a polyfunctional (meth) acrylic ester monomer (component f).

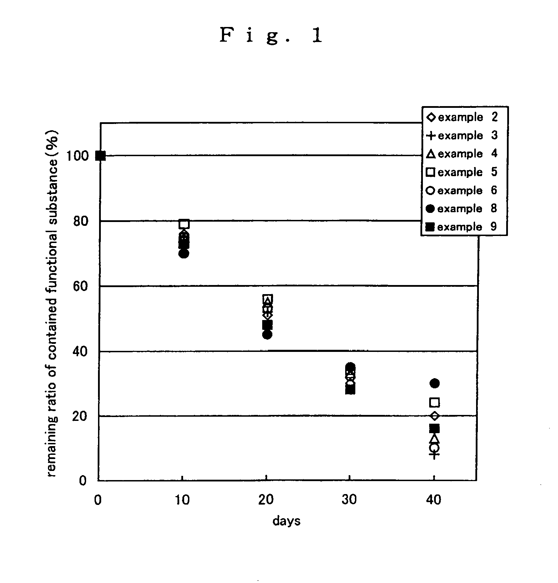

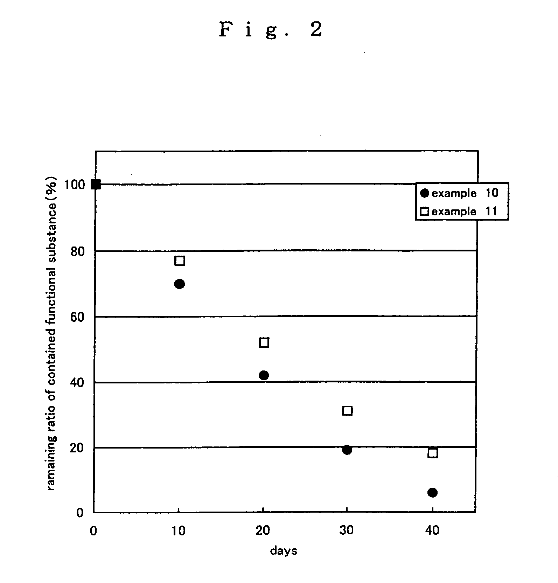

Fig. 1 and Fig. 2 are graphs showing release state of functional substances at the preparations of working examples.

In the following, this invention will be described in detail.

Generally, IPN indicates the material composed of interlaced two polymers being formedwhen onepolymer is polymerizedor cross-linked under the existence of another polymer network.. Micro-cells are formed in openings in the interlaced two polymers.

An aqueous dispersion-type sustained release preparation according to this invention is in the form of IPN structure composed of microgel A and microgel B, and containing a functional substance dispersed in micro-cells in the structure. The functional substance is going to be released from these micro-cells at a constant release speed out of these particles.

Thus, those components, such as a functional substance, a monomer having good affinity to the functional substance and a polyfunctional monomer are essential components for microgel A, whereas a monomer having poor affinity to the functional substance and a polyfunctional monomer are essential for microgel B, and the units composed such monomers control the sustained releasability.

The monomer forming microgel A and having good affinity to a functional substance serves to keep the functional substance stably in the microgel, thus, in case a monomer having poor affinity to the functional substance is used, especially in polymerization process, stabilized emulsion cannot be obtained. As a judgmental standard of the af finity of amonomer to a functional substance, studying a dispersion state of a mixed solution would be useful. The mixed solution can be prepared by mixing a polymer component (having a weight average molecular weight of 5,000 or more) and a functional substance at the ratio of 1:9 by weight, wherein the said polymer component has been obtained by usual polymerization process such as solution polymerization using the subject monomer component. For example, if the polymer component is easily dissolved in the functional substance, it indicates the monomer component has good affinity to the functional substance, and on the contrary, being hardly dissolved indicates poor affinity.

As mentioned above, (meth)acrylic ester monomers (component b) are used because they easily make a stabilized emulsion and from the standpoint of getting a higher polymerization conversion rate. Still, other monomer components can be contained in the monomer components forming microgel A as far as not to interfere with the functions of microgel A.

Concrete examples of component b are (meth)acrylic ester monomers having an alkyl chain with 1-24 carbon atoms like methyl(meth)acrylate, ethyl(meth)acrylate, propyl(meth)acrylate, butyl(meth)acrylate, pentyl(meth)acrylate, hexyl(meth)acrylate, n-octyl(meth)acrylate, 2-etylhexyl(meth)acrylate, lauryl(meth)acrylate, cetyl(meth)acrylate, stearyl(meth)acrylate, behenyl(meth)acrylate, etc.

In case pheromones are used as a functional substance, (meth) acrylic ester monomers having an alkyl chain with 6-20 carbon atoms are preferable to have good affinity to the pheromones. As concrete examples, hexyl(meth)acrylate, n-octyl(meth)acrylate, 2-ethylhexyl(meth)acrylate, lauryl(meth)acrylate, cetyl(meth)acrylate, stearyl(meth)acrylate can be named. (Meth)acrylic ester monomers having an alkyl chain with 8-20 carbon atoms are still more preferable, thus, n-octyl(meth)acrylate, 2-ethylhexyl(meth)acrylate, lauryl(meth)acrylate, cetyl(meth)acrylate, stearyl(meth)acrylate can be named as concrete examples.

On the other hand, the monomer forming microgel B and having poor affinity to a functional substance is a component that is used for purposes of releasing the functional substance and giving adhesivity to the particles. Thus, a monomer differing from the ones having good affinity mentioned above should be used. Furthermore, for this monomer having poor affinity, it is necessary to choose the one which can swell microgel A. If it can not swell microgel A, polymerization will proceed outside of microgel A and IPN will not possibly be formed. Thus, as a judgmental standard to choose a monomer having poor affinity, the above-described judgmental standard of affinity of a monomer to a functional substance should be applied first, and then, further judged by studying a dispersion state of another mixed solution. This mixed solution can be prepared by mixing a polymer component (having a weight average molecular weight of 5,000 or more) and a subject monomer expected to have poor affinity at the ratio of 1:9 by weight, wherein the said polymer component has been obtained by solution polymerization etc. using the monomer having good affinity. For example, if the subject monomer can dissolve or disperse the polymer component, it can be utilized as a monomer forming microgel B, and if cannot dissolve at all, it is judged not to be able to form IPN and cannot be utilized.

As described above, any monomer can be used as long as it has poor affinity to the functional substance, however, (meth)acrylic ester monomers (component e) are preferably used because they would make the control of adhesivity of the particles easier and from the standpoint of getting a higher polymerization conversion rate. Still, other monomer components can be contained in the monomer components forming microgel B as far as not to interfere with the functions of microgel B.

Concrete examples of component e are (meth)acrylic ester monomers having an alkyl chain with 1-24 carbon atoms, for example, methyl (meth) acrylate, ethyl (meth)acrylate, propyl(meth)acrylate, butyl(meth)acrylate, pentyl(meth)acrylate, hexyl(meth)acrylate, n-octyl(meth)acrylate, 2-etylhexyl(meth)acrylate, lauryl(meth)acrylate, cetyl(meth)acrylate, stearyl(meth)acrylate, behenyl(meth)acrylate, etc.

In case pheromones are used as a functional substance, those having an alkyl chain with 1-8 carbon atoms are preferable. As concrete examples, methyl(meth)acrylate, ethyl(meth)acrylate, propyl(meth)acrylate, butyl(meth)acrylate, pentyl(meth)acrylate, hexyl(meth)acrylate, n-octyl (meth)acrylate, 2-etylhexyl (meth)acrylate are named. More preferably, those which have an alkyl chain with 1-6 carbon atoms are used. As concrete examples, methyl(meth)acrylate, ethyl(meth)acrylate, propyl(meth)acrylate, butyl(meth)acrylate, pentyl(meth)acrylate, hexyl(meth)acrylate are named.

As a further essential component forming microgel A and microgel B, polyfunctional monomer is used. Any monomer can be used as long as having 2 or more radical polymerizing functional groups such as vinyl groups, however, using polyfunctional (meth)acrylic ester monomers (component c: used for microgel A, component f: used for microgel B) enables to control excellent sustained releasability. Component c is mandatorily represented by polyfunctional (meth) acrylic ester monomers.

This monomer crosslinks the functional substance and the monomer having good affinity or the monomer having poor affinity to form a gel, and forms microcells at IPN formation. Thus, sustained releasability can be controlled depending on the amounts of polyfunctional monomers used. Basically, using larger amounts of polyfunctional monomers results in finer microcells and slower sustained releasability, and using smaller amounts of polyfunctional monomers results in coarser microcells and faster sustained releasability.

Concrete examples of component c and component f are, for example, ethylene glycol di(meth)acrylate, diethylene glycol di(meth)acrylate, triethylene glycol di (meth) acrylate, polyethylene glycol di (meth) acrylate, 1,3-butylene glycol di(meth)acrylate, 1,4-butanediol di(meth)acrylate, 1,6-hexanediol di(meth)acrylate, neopentyl glycol di(meth)acrylate, diacryl phthalate, allyl (meth)acrylate, 2-hydroxy-1,3-di(meth)acryloxy propane, 2,2-bis(4-((meth)acryloxyethoxy)phenyl)propane, trimethylolpropanetri(meth)acrylate, tetramethylolmethane(meth)acrylate, pentaerythritoltri(meth)acrylate, dipentaerythritoltri(meth)acrylate, pentaerythritolpenta(meth)acrylate, dipentaerythritolhexa(meth)acrylate, tetramethylolmethanetetra(meth)acrylate, etc.

To achieve excellent sustained releasability, ethyleneglycoldi(meth)acrylate, diethyleneglycoldi(meth)acrylate, triethyleneglycoldi(meth)acrylate, polyethyleneglycoldi(meth)acrylate, 1,3-butyleneglycoldi(meth)acrylate, 1,4-butanedioldi(meth)acrylate, 1,6-hexanedioldi(meth)acrylate, neopentylglycoldi(meth)acrylate, diacrylphthalate, allyl(meth)acrylate, trimethylolpropanetri(meth)acrylate, pentaerythritoltri(meth)acrylate, dipentaerythritoltri(meth)acrylate, pentaerythritolpenta(meth)acrylate, tetramethylolmethanetetra(meth)acrylate, etc. are preferable.

As for the said component c and component f, either one kind of monomer or combination of 2 or more kinds of monomers may be used for each of them.

Further, in the IPN in which microgel A and microgel B are chemically bonded, IPN ratio in particles increases comparing to in the IPN not chemically bonded, which also means the increase of microcells. That causes uniform dispersion of functional substance in the microcells and tends to result in better aqueous dispersion stability of particles and better control of sustained releasability. The IPN ratio is increased because a source of the radicals which work at the formation of microgel B exists in microgel A so that IPN is formed efficiently.

To form the chemically bonded IPN, either a (meth) acrylic ester monomer having a photo-initiating group (component a1) which works as a photo polymerization initiator or a radical polymerizing organic peroxide (component a2) which works as a organic peroxide is essential in microgel A.

These monomers have a polymerizing group to synthesize microgel B, thus, at the formation of microgel A, polymerization has to be proceeded paying attention not to decompose the polymerizing group and generate radicals.

For example, in case a (meth)acrylic ester monomer having a photo-initiating group is used, careful light shielding is necessary at polymerization to synthesize microgel A. In case a radical polymerizing organic peroxide is used, polymerization temperature has to be kept 90°C or less, preferably 80°C or less.

As component a1, a (meth)acrylic ester monomer having a photo-initiating group, for example, 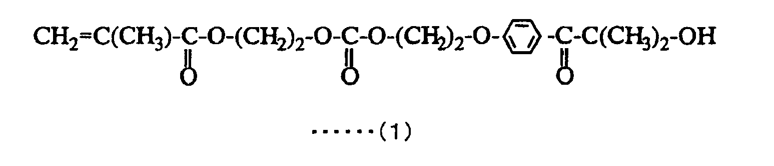 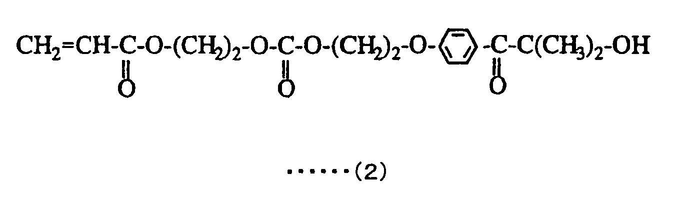 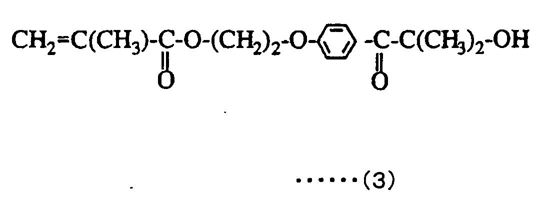 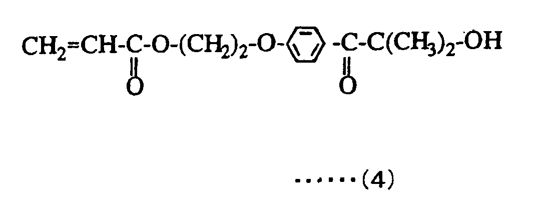 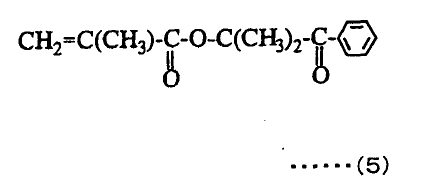 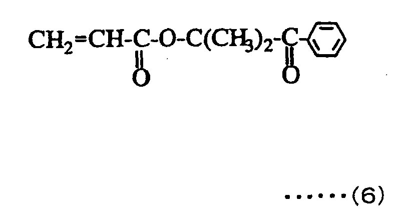 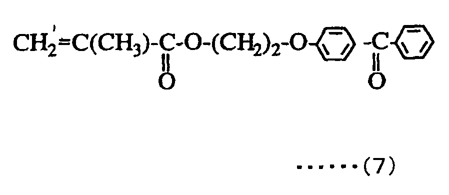 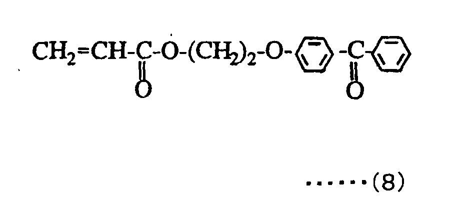 etc. can be used.

Among them, 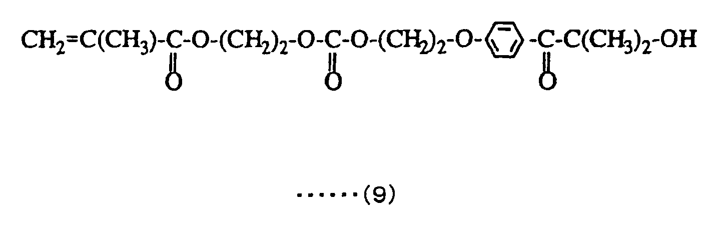 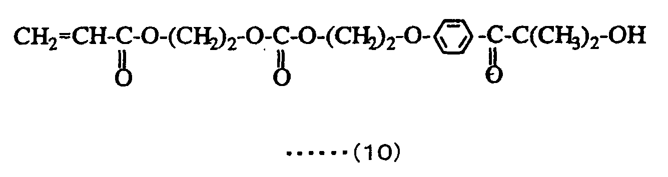 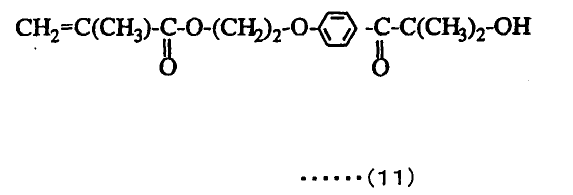 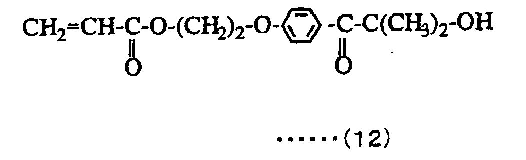 etc. are preferably used from the standpoint of better IPN formation.

In the IPN obtained by using these (meth) acrylic ester monomers having a photo-initiating group, IPN is efficiently formed so that high levels of aqueous dispersion stability of particles and constant sustained releasability are achieved. Furthermore, IPN can be formed in a very short time because microgel B is synthesized by photo-initiated polymerization.

Next, concrete examples of radical polymerizing organic peroxide (component a2) are, for example, t-butylperoxyacryloyloxyethyl carbonate, t-butylperoxymethacryloyloxyethyl carbonate, t-butylperoxyallyl carbonate, t-butylperoxymethacryl carbonate, etc.

Among them, t-butylperoxyacryloyloxyethyl carbonate, t-butylperoxymethacryloyloxyethyl carbonate, etc. are preferably used from the standpoint of IPN formation and higher decomposition temperatures of their peroxy bond.

Further, a hydrophilic monomer (component d), which can be dissolved in water at any ratio, can be copolymerized in microgel A to improve stability of the emulsion. The hydrophilic component is oriented on the surface of polymers (particles) and enables to improve their aqueous dispersibility.

Concrete examples of component d are, for example, unsaturated mono carboxylic acid monomers like (meth)acrylic acid, itaconic acid, crotonic acid, fumaric acid, maleic acid, mesaconic acid, citraconic acid and β -(meth)acryloyloxyethylhydrogen succinate, etc., or unsaturated acid anhydride monomers like maleic anhydride, itaconic anhydride, citraconic anhydride, and 4-methacryloxyethyltrimellitic anhydride etc., or monomers having a phenol group like hydroxyphenoxyethyl(meth)acrylate, hydroxyphenoxypolyethylene glycol (meth) acrylates having 2-90 moles of adduct of ethyleneoxide, hydroxyphenoxypolypropropylene glycol(meth)acrylates having 2-90 moles of adduct propyleneoxides, vinyl phenol, hydroxyphenylmaleimide etc., or monomers having a sulfonic acid group like sulfoxyethyl(meth)acrylate, styrenesulfonate, acrylamide-t-butylsulfonate, (meth)allylsulfonic acid etc., or monomers having a phosphoric acid group like mono-2-(meth)acryloyloxyethyl acid phosphate etc., or (meth)acrylamide monomers like N,N-dimethyl(meth)acrylamide, N,N-diethyl(meth)acrylamide, N-isopropyl(meth)acrylamide, acryloylmorpholine, N,N-dimethylaminopropyl(meth)acrylamide, (meth)acrylamide and N-methylol (meth) acrylamide etc., or aminoalkyl (meth) acrylate monomers like N,N-diethylaminoethyl(meth)acrylate and N,N-dimethylaminoethyl(meth)acrylate etc., or hydroxyalkyl(meth)acrylate monomers like 2-hydroxyethyl(meth) acrylate, 2-hydroxypropyl(meth)acrylate and 2,3-dihydroxypropyl(meth)acrylate etc., or mono (meth)acrylate monomers of polyoxyethylene like polyethylene glycol mono (meth)acrylates having 2-98 moles of adduct ethyleneoxides, methoxypolyethylene glycol mono(meth)acrylates having 2-98 moles of adduct ethyleneoxides, phenoxypolyethylene glycol mono(meth)acrylate having 2-98 moles of adduct ethyleneoxides, nonylphenol monoethoxylate (meth) acrylates having 1-4 moles of adduct ethyleneoxides, methoxyethylacrylate and ethoxydiethylene glycol (meth)acrylate etc., or monomers having an acid group like sodium (meth)acrylate, sodium styrenesulfonate, sodium sulfonate ethoxy (meth)acrylate and sodium acrylamide-t-butylsulfonate etc., or (meth)acrylate monomers having a tetravalent ammonium salt group like (meth)acryloylhydroxyethyltrimethylammonium chloride and (meth)acryloylhydroxypropyltrimethylammonium chloride etc., or (meth)allyl compound monomers like allyl glycol, polyethylene glycol mono(meth)allyl ether having 2-32 moles of adduct ethyleneoxides, methoxypolyethylene glycol monoallyl ether etc., or compound monomers having a heterocyclic ring like N-vinylpyrrolidone and N-vinylcaprolactam etc., or vinyl cyanide monomers like acrylonitrile, methacrylonitrile and vinylidene cyanide etc.

To give the microgel excellent aqueous dispersibility, unsaturated monocarboxylicacidmonomerslike (meth)acrylicacid, itaconicacid, crotonic acid, fumaric acid ,maleic acid, mesaconic acid, citraconic acid and β - (meth) acryloyloxyethylhydrogen succinate etc., or monomers having a phenol group like hydroxyphenoxyethyl(meth)acrylate, hydroxyphenoxypolyethylene glycol(meth)acrylates having 2-40 moles of adduct of ethyleneoxides, hydroxyphenoxypolypropropylene glycol(meth)acrylates having 2-40 moles of adduct of propyleneoxides, vinyl phenol, hydroxyphenylmaleimide etc., or monomers having a sulfonic acid group like sulfoxyethyl(meth)acrylate, styrenesulfonate, acrylamide-t-butylsulfonate, (meth)allylsulfonic acid etc., or (meth)acrylamide monomers like N,N-dimethyl(meth)acrylamide, N,N-diethyl(meth)acrylamide, N-isopropyl(meth)acrylamide, acryloylmorpholine, N,N-dimethylaminopropyl(meth)acrylamide, (meth)acrylamide and N-methylol(meth)acrylamide etc.,or hydroxyalkyl(meth)acrylate monomers like 2-hydroxyethyl(meth)acrylate, 2-hydroxypropyl(meth)acrylate and 2,3-dihydroxypropyl(meth)acrylate etc., or mono(meth)acrylate monomers of polyoxyethylene like polyethylene glycol mono(meth)acrylates having 2-98 moles of adduct of ethyleneoxides, methoxypolyethylene glycol mono (meth) acrylates having 2-98 moles of adduct ethyleneoxides, phenoxypolyethylene glycol mono(meth)acrylate having 2-98 moles of adduct of ethyleneoxides, nonylphenol monoethoxylate (meth) acrylates having 1-4 moles of adduct ethyleneoxides, methoxyethylacrylate and ethoxydiethylene glycol (meth)acrylate etc., or (meth)acrylate monomers having a tetravalent ammonium salt group like (meth)acryloylhydroxyethyltrimethylammonium chloride and (meth) acryloylhydroxypropyltrimethylammonium chloride etc., or (meth) allyl compound monomers like allyl glycol, polyethylene glycol mono(meth)allyl ether having 3-15 moles of adduct of ethyleneoxides, methoxypolyethylene glycol monoallyl ether etc., or compound monomers having a heterocyclic ring like N-vinylpyrrolidone and N-vinylcaprolactam etc., or vinyl cyanide monomers like acrylonitrile, methacrylonitrile and vinylidene cyanide etc. are preferably named.

The aqueous dispersion-type sustained release preparation containing functional substance of this invention can be used in the either form of an emulsion of aqueous dispersion state, as a powder obtained by drying the said emulsion, or as a film obtained from the same . As the means to obtain sustained release preparations in dried state from those in aqueous dispersion state, any ordinary means of eliminating water can be used, for example, vacuum drying, freeze drying etc. can be employed to produce the preparations.

The solid ratio (total amount of microgel A and microgel B) in aqueous dispersion state would usually be 1-60 wt%, preferably 10-50 wt%, and the average particle size of sustained release preparations dispersed in water would usually be 10-1000 nm, preferably 50-700 nm.

Besides, to improve the aqueous dispersibility of the sustained release preparation and to prevent denaturation of the functional substance, pH of the emulsion can optionally be adjusted by an alkaline substance such as ammonia water or sodium hydrate. In the case, the pH value range would usually be 2-9, preferably 3-8, more preferably 3.5-7.5. If the pH value deviates from the above-mentioned range, stability of the emulsion will be lost.

As for the said monomer components forming microgel A, either one kind of monomer or combination of 2 or more kind of monomers may be used for the each component.

Still, in the said monomer components forming microgel A, other monomer component than the said monomers, for example, aromatic vinyl monomers like styrene, α-methylstyrene and vinyltoluene etc., as well as vinyl ester monomers like vinyl acetate, vinylpropionic acid, vinyl butyrate, vinyl pivalate, vinyl laurate and versatic acid vinyl ester etc. can be used as far as not to interfere with the functions of microgel A.

Kind and ratio of the said components used at the formation of microgel A by emulsion copolymerization can properly be changed depending on the functional substance intended to be contained.

The ratio of component a1 or component a2 used based on the total amount of monomer components of microgel A would usually be 0-20 wt%, preferably 0.05-15 wt%. If it becomes more than 20 wt%, radical decomposition or excessive gelation will possibly be induced at the reaction by a photo-initiating group or a peroxy bond introduced in the obtained microgel.

On the other hand, the ratio of component b used, based on the total amount of monomer components of microgel A, would usually be 45-99.95 wt%, preferably 50-95 wt%. If it is less than 45 wt%, the functional substance will not be stably contained in the sustained release preparation, and more than 99 wt%, the emulsion will become unstable and coagulation tends to easily be generated.

Further, the ratio of component c used, based on the total amount of monomer components of microgel A, would usually be 0.05-15 wt%, preferably 0.2-10 wt%. If it is less than 0.05 wt%, the sustained release speed will become too fast, and more than 15 wt%, sustained releasability tends not to be exhibited.

Still further, the ratio of component d used, based on the total amount of monomer components of microgel A, would usually be 0-45 wt%, preferably be 5-40 wt%. If it becomes more than 45 wt%, the functional substance tends not to be stably contained in the sustained release preparation.

The ratio of component e used, based on the total amount of monomer components of microgel B, would usually be 60-99.9 wt%, preferably be 70-99 wt%.

Further, the ratio of component f used, based on the total amount of component e and component f, would usually be 0.1-40 wt%, preferably be 1-30 wt%.

If the ratio deviates from the above-mentioned range, additional functions such as excellent sustained releasability and adhesivity etc. tends not to be easily achieved.

The ratio of microgel B to microgel A would usually be 10-500 parts by weight, preferably be 20-40 parts by weight towards 100 parts by weight of microgel A. If the ratio deviates from 10-500 parts by weight, formation of the IPN with a dense and uniform inter penetrating networks will become difficult so that it is unpreferable for a sustained release preparation.

The IPN type sustained release preparation formed by microgel A and microgel B differs from other ordinary microgels in showing excellent sustained releasability being able to keep the release amount at the later stage almost as much as the one at the beginning.

Examples for the said functional substance are listed below; they are pheromones like 14-methyl-1-octatadecene, Z9-tricosene, E4-tridecenyl acetate, dodecyl acetate, Z7-dodecenyl acetate, Z8-dodecenyl acetate, Z9-dodecenyl acetate, E7,E9-dodecadienyl acetate, Z9-tetradecenyl acetate, Ell-tetradecenyl acetate, Z11-tetradecenyl acetate, Z9,E11-tetradecadienyl acetate, Z9,E12-tetradecadienyl acetate, Z11-hexadecenyl acetate, Z7,Z/E11-hexadecadienyl acetate, Z13-hexadecatrienyl acetate, Z13-octadecenyl acetate, E13,Z13-octadecadienyl acetate, Z11-hexadecenal, Z13-octadecenal, Z13-icosen-10-on, 7,8-epoxy-2-methyloctadecane and 8-methyl-2-decylpropionate etc., or medicines like ampicillin, sodium salicylate, sulpyrine, sodium indomethacin, morphine hydrochloride, ephedrine Hydrochloride, noscapin hydrochloride, chlorpromazine hydrochloricde and hexamethonium bromide etc., or agricultural chemicals like fenobucarb, carbosulfan, benfuracarb, cyhalothrin, fenvalerate, fenitrothion, profenofos, ethylthiometon, diazinon, etrimfos and edifenphos etc., or fragment materials based on rose, jasmine, gardenia, fragrant olives and citrus.

To achieve excellent sustained releasability, pheromones like 14-methyl-1-octatadecene, Z9-tricosene, E4-tridecenyl acetate, dodecyl acetate, Z7-dodecenyl acetate, Z8-dodecenyl acetate, Z9-dodecenyl acetate, E7,E9-dodecadienyl acetate, Z9-tetradecenyl acetate, E11-tetradecenyl acetate, Z11-tetradecenyl acetate, Z9,E11-tetradecadienyl acetate, Z9,E12-tetradecadienyl acetate, Z11-hexadecenyl acetate, Z7,Z/E11-hexadecadienyl acetate, Z13-hexadecatrienyl acetate, Z13-octadecenyl acetate, E13,Z13-octadecadienyl acetate, Z11-hexadecenal, Z13-octadecenal, Z13-icosen-10-on, 7,8-epoxy-2-methyloctadecane and 8-methyl-2-decylpropionate etc., or agricultural chemicals like fenobucarb, carbosulfan, benfuracarb, cyhalothrin, fenvalerate, fenitrothion, profenofos, ethylthiometon, diazinon, etrimfos and edifenphos etc. are preferably named.

These functional substances may be used either singularly or in combination.

Further, the content of these functional substance would usually be 0.1-150 parts by weight, preferably be 5-100 parts by weight towards 100 parts by weight of microgel A. If it is less than 0.1 parts by weight, the sustained releasability will become unsufficient, and more than 150 parts by weight, an aqueous dispersibility of the microgel will become poorer.

Hereinbelow, the method for producing an aqueous dispersion-type sustained release preparation containing a functional substance inside of its IPN formed by 2 steps will be described.

The producing method can be carried out in 2 ways. Monomers named here are typical ones described only for illustrative purpose, still, are not intended to limit this invention.

Firstly, the case in which a (meth) acrylic ester monomer (component a1) having photo-initiating group is used will be described.

In a first step, microgel A having photo-initiating group is obtained by emulsion copolymerization.

The emulsion copolymerization is carried out using a monomer component comprising a (meth)acrylic ester monomer having a photo-initiating group (component a1), a (meth) acrylic ester monomer (component b), a polyfunctional (meth) acrylic ester monomer (component c) and optionally a hydrophilicmonomer (component d) as well as a functional substance, a surface active agent, a polymerization initiator and water to obtain a emulsion.

Hereinafter, a preferable process for the first step will be described more concretely. At first, a monomer component comprising a (meth)acrylic ester monomer (component a1), a (meth) acrylic ester monomer (component b), a polyfunctional (meth)acrylic ester monomer (component c) and optionally a hydrophilic monomer (component d) as well as a functional substance, a surface active agent and water are stirred and pre-emulsified using a stirrer with high shear strain like homo-mixer, to be uniformly emulsified and dispersed. Then, the obtained pre-emulsion is dropped into water containing a polymerization initiator and emulsion copolymerized. In this operation, the functional substance is taken into microgel A with accompanying proceeding of the polymerization. In this procedure, a polymerization initiator can be previously added at the preparation of the pre-emulsion.

The said surface active agent is not limited to any special types and all the surface active agents can be used.

These surface active agents may be used either singularly or in combination of 2 or more kinds, and further, the content of the surface active agent would usually be 0.1-25 parts by weight, preferably be 0.5-20 parts by weight towards 100 parts by weight of total amount of monomers used in the first step. If the content is less than 0.1 parts by weight, the emulsion will become unstable and coagulations will be generated, and more than 25 parts by weight, the viscosity of the emulsion tends to become higher.

Still, in this invention, the use of anionic surface active agents or nonionic surface active agents among surface active agents are especially preferable.

The polymerization initiator is not limited to any special types and those listed below can be used.

The amount of the polymerization initiator used would usually be 0.05-10 parts by weight, preferably be 0.1-5 parts by weight towards 100 parts by weight of total amount of monomers used in the first step. If it is less than 0.05 parts by weight, the polymerization initiation ability will become poorer, and more than 10 parts by weight, the polymerization stability tends to become poorer.

The polymerization temperature at the said emulsion polymerization would usually be 40-120°C, preferably 60-100°C, while the polymerization duration would usually be 2-12 hours, preferably 4-10 hours.

In a second step, microgel B is formed onto microgel A. In other words, a (meth)acrylic ester monomer (component e) and a polyfunctional (meth)acrylic ester monomer(component f) are dropped into the said emulsion of microgel A including a functional substance obtained in the first step and polymerized by irradiation of activated energy lines.

Secondly, the case in which a radical-polymerizing organic peroxide (component a2) is used will be described.

In a first step, microgel A having a peroxide bond is obtained by emulsion copolymerization.

The emulsion copolymerization is carried out using a monomer component comprising a radical-polymerizing organic peroxide (component a2), a (meth)acrylic ester monomer (component b), a polyfunctional (meth)acrylic ester monomer (component c) and optionally a hydrophilic monomer (component d) as well as a functional substance, a surface active agent, a polymerization initiator and water to obtain a emulsion.

Hereinafter, a preferable process for the first step will be described more concretely. At first, a monomer component comprising a radical-polymerizing organic peroxide (component a2), a (meth) acrylic ester monomer (component b), a polyfunctional (meth)acrylic ester monomer (component c) and optionally a hydrophilic monomer (component d) as well as a functional substance, a surface active agent and water are stirred and pre-emulsified using a stirrer with high shear strain like homo-mixer, to be uniformly emulsified and dispersed. Then, the obtainedpre-emulsion is dropped into water containing a polymerization initiator and emulsion copolymerized. In this operation, the functional substance is taken into microgel A with accompanying proceeding of the polymerization. In this procedure, a polymerization initiator can be previously added at the preparation of the pre-emulsion.

The said surface active agent is not limited to any special types and all the surface active agents can be used.

These surface active agents may be used either singularly or in combination of 2 or more kinds, and further, the content of the surface active agent would usually be 0.1-25 parts by weight, preferably be 0.5-20 parts by weight towards 100 parts by weight of total amount of monomers used in the first step. If the content is less than 0.1 parts by weight, the emulsion will become unstable and coagulations will be generated, and more than 25 parts by weight, the viscosity of the emulsion tends to become higher.

The said polymerization initiator is not limited to any special types and those listed below can be used.

The amount of the polymerization initiator used would usually be 0.05-10 parts by weight, preferably be 0.1-5 parts by weight towards 100 parts by weight of total amount of monomers used in the first step. If it is less than 0.05 parts by weight, the polymerization initiation ability will become poorer, and more than 10 parts by weight, the polymerization stability tends to become poorer.

The polymerization temperature at the said emulsion polymerization would usually be 40-90°C, preferably be 60-80°C, while the polymerization duration would usually be 2-12 hours, preferably be 4-10 hours.

In a second step, microgel B is formed onto microgel A. In other words, a (meth) acrylic ester monomer (component e) and a polyfunctional (meth) acrylic ester monomer(component f) are dropped into the said emulsion of microgel A including a functional substance obtained in the first step and polymerized by peroxide bonds of microgel A. Almost all the peroxide bonds would remain through the formation of microgel A because the 10 hour half-value decay temperature for them are 90°C or more.

Thus, reaction conditions for the second step are as follows: in a pressure durable reactor vessel like autoclave; the polymerization temperature would be 80-180°C, preferably be 110-150°C; the polymerization duration would usually be 2-12 hours, preferably be 4-10 hours.

Concrete examples for the surface active agent will be listed below.

For anionic surface active agents, alkyl sulfates of alkali metals like sodium dodecyl sulfate or potassium dodecyl sulfate etc., ammonium alkyl sulfates like ammonium dodecyl sulfate etc., sodium dodecylpolyglycol ether sulfate, ether sulfate, alkyl sulfonates like alkali metal salts of sulfonated paraffin or ammonium salts of sulfonated paraffin, fatty acid salts like sodium laurate or triethanolamine oleate or triethanolamine abietate etc., alkyl allyl sulfonates like sodium dodecylbenzene sulfonate or alkali metal salt sulfates of alkali phenol hydroxy ethylene etc., dialkyl sulfosuccinates like highly alkylated naphthalenesulfonate, naphthalene sulfonate formaldehyde polycondensation and sodium dioctyl sulfosuccinate etc., polyoxyethylene alkyl ether sulfate salts, polyoxyethylene alkylphenyl ether sulfate salts, or polyoxyethylene alkylallyl sulfate salts etc. can be named as examples.

For nonionic surface active agents, polyoxyethylene alkyl ethers, polyoxyethylene alkylallyl ethers, sorbitan fatty acid esters, polyoxyethylene sorbitan fatty acid esters, fatty acid monoglyceride like glycerol monolaurate, polyoxyethylene-oxypropylene copolymer, or polycondensation products of ethyleneoxide and fatty acid amines or amides or acids can be named as examples.

For cationic surface active agents, octadecylamine acetate, dodecyltrimethylammonium chloride, hexadecyltrimethylammonium chloride, tetradecyldimethylbenzylammonium chloride, octadecyldimethylbenzylammonium chloride, or dioleyldimethylammonium chloride etc. can be named as examples.

For ampholytic surface active agents, dimethyllauryl betaine, sodium lauryldiaminoethylglycin, amidebetaine type, or imidazoline type etc. can be named as examples.

For polymer surface active agents, water-soluble polymers like polyvinyl alcohol, sodium poly(meth)acrylate, potassium poly(meth)acrylate, ammonium poly(meth)acrylate, hydroxyethyl (meth)acrylate polymer and hydroxypropyl (meth)acrylate polymer etc. can be named as examples.

For reactive emulsifying agents, "LATEMUL" S-180 or S-180A available from Kao Corporation, "HITENOL" RN series or HS series or "NEW FRONTIER" A-229E or N-177E available from DAI-ICHI KOGYO SEIYAKU CO., Ltd., or "Antox" MS-60 or MS-2N or RA-1120 or RA-2614 or RMA-564 or RMA-569 or RMA-1114 available from Nippon Nyukazai Co., Ltd., or "ADEKA REASOPE" NE-10 or NE-20 or NE-40 available from Asahi Denka Kogyo K.K., or "NK ester" M20G or M40G or M-90G or M-230G available from SHIN NAKAMURA CHEMICAL CO., LTD. etc. can be named as examples.

The said polymerization initiator is not limited to any special types and sodium persulfate, potassium persulfate, ammonium persulfate, acetyl peroxide, isobutyl peroxide, octanoyl peroxide, decanoyl peroxide, lauroyl peroxide, 3,3,5-trimethylhexanoyl peroxide, benzoyl peroxide, diisopropyl peroxydicarbonate, tertiarybutyl peroxyacetate, tertiarybutyl peroxymaleate, 2,2-azobis(isobutyronitrile), 2,2-azobis(2-methylbutyronitrile), 2,2-azobis(2,4-dimethylvaleronitrile), 2,2-azobis(4-methoxy-2,4-dimethylvaleronitrile), 1,1-azobis(cyclohexane-1-carbonitrile), 2-(carbamoylazo)isobutyronitrile, 1,1-azobis{2-[N-(4-chlorophenyl)amidino]propane} dihydrochloride, 2,2-azobis[2-(N-phenylamidino)propane] dihydrochloride, 2,2-azobis{2-[N-(4-hydroxy phenyl)amidino]propane} dihydrochloride, 2,2-azobis[2-(N-benzylamidino)propane] dihydrochloride, 2,2-azobis[2-(N-allylamidino)propane] dihydrochloride, 2,2-azobis(2-amidinopropane) dihydrochloride, 2,2-azobis{2-[N-(2-hydroxyethyl)amidino]propane} dihydrochloride, 2,2-azobis[2-(5-methyl-2-imidazoline-2-yl)propane] dihydrochloride, 2,2-azobis[2-(2-imidazoline-2-yl)propane] dihydrochloride, 2,2-azobis[2-(4,5,6,7-tetrahydro-1H-1,3-diacepine-2-yl)propane] dihydrochloride, 2,2-azobis[2-(3,4,5,6-tetrahydropyrimidine-2-yl)propane] dihydrochloride, 2,2-azobis[2-(5-hydroxy-3,4,5,6-tetrahydropyrimidine-2-yl)propane] dihydrochloride, 2,2-azobis{2-[1-(2-hydroxyethyl)-2-imidazoline-2-yl]propane} dihydrochloride, 2,2-azobis[2-(2-imidazoline-2-yl) propane], 2,2-azobis{2-methyl-N-[1,1-bis(hydroxymethyl)-2-hydroxyethyl]propionami de}, 2,2-azobis[2-methyl-N-(2-hydroxyethyl)-propionamide], 2,2-azobis{2-methyl-N-[1,1-bis(hydroxymethyl)ethyl] propionamide}, 2,2-azobis(2-methylpropionamide) dihydrate, 4,4'-azobis(4-cyanovaleric acid), 2,2-azobis[2-(hydroxymethyl) propionitrile] etc. can be named as examples.

To achieve an excellent polymerization stability, preferably, sodium persulfate, potassium persulfate, ammonium persulfate, acetyl peroxide, isobutyl peroxide, 3,3,5-trimethylhexanoyl peroxide, benzoyl peroxide, diisopropyl peroxydicarbonate, tertiarybutyl peroxymaleic acid, 2,2-azobis(2-methylbutyronitrile),2-(carbamoylazo)isobutyronitrile, 2,2-azobis[2-(N-phenylamidino)propane] dihydrochloride, 2,2-azobis{2-[N-(4-hydroxyphenyl)amidino]propane} dihydrochloride, 2,2-azobis[2-(N-benzylamidino)propane] dihydrochloride, 2,2-azobis(2-amidino propane) dihydrochloride, 2,2-azobis{2-[N-(2-hydroxyethyl)amidino]propane} dihydrochloride, 2,2-azobis[2-(2-imidazoline-2-yl)propane] dihydrochloride, 2,2-azobis[2-(4,5,6,7-tetrahydro-1H-1,3-diacepine-2-yl)propane] dihydrochloride, 2,2-azobis[2-(3,4,5,6-tetrahydropyrimidine-2-yl)propane] dihydrochloride, 2,2-azobis{2-[1-(2-hydroxyethyl)-2-imidazoline-2-yl]propane}dihydrochlo ride, 2,2-azobis[2-(2-imidazoline-2-yl) propane], 2,2-azobis[2-methyl-N-(2-hydroxyethyl)-propionamide], 2,2-azobis{2-methyl-N-[1,1-bis(hydroxymethyl)-2-hydroxyethyl]propionami de}, 4,4'-azobis(4-cyanovaleric acid), 2,2-azobis[2-(hydroxymethyl)propionitrile] etc. can be named as examples.

Hereinbelow, this invention will be described in terms of preferred embodiments though this invention is not limited by the embodiments shown below.

### <Example 1>

60 parts by weight of ion-exchanged water was placed into a flask equipped with a stirrer, a thermometer, a cooler, a drip funnel and a nitrogen gas tube, and heated up to 80 °C under agitation and nitrogen gas flow. Then, 0.4 parts by weight of potassium persulfate was added.

4 parts by weight of Z11-hexadecenyl acetate (sex pheromone of *Plutella xylostella Linnaeus* (diamondback moth)) as a functional substance, totally 21 parts by weight of monomer components (composition ratio is shown in Table 1), 1 part of sodium dodecyl sulfate (an anionic surface active agent) and 19 parts by weight of ion-exchanged water were placed into a beaker and stirred by a homo-mixer for 15 minutes at 10,000 rpm at ambient temperature to prepare a pre-emulsion.

Into the said flask, the pre-emulsion was dropped gradually taking 3 hours under the kept temperature condition of 80 °C, then further polymerized for 4 hours to obtain a emulsion (aqueous dispersion-type microgel A containing Z11-hexadecenyl acetate).

Totally 8 parts by weight of monomer components e and f previously mixed at composition ratio shown in Table 2 were dropped onto the obtained emulsion and radical polymerization reaction was proceeded for 1 hour under the irradiation of activated energy line to obtain an aqueous dispersion-type sustained release preparation containing Z11-hexadecenyl acetate in its IPN. Completion of the polymerization was confirmed by GC analysis detecting any remainingmonomers in the emulsion. Then, to improve the aqueous dispersibility of the sustained release preparation, ammonia water was added and the pH of the emulsion was adjusted to 7. The average particle size measured by dynamic light scattering method (DLS) was 130 nm.

To evaluate the aqueous dispersibility of the sustained release preparation, the emulsion was placed still in a temperature controlled bath at 25 °C and was studied deposition of coagulations. The result is shown in Table 3. The pH value for the emulsion is also shown in Table 3.

Further, to investigate its sustained releasability, the obtained aqueous dispersion-type sustained release preparation was dried to obtain a sustained release preparation containing Z11-hexadecenyl acetate in powder form.

Next, this sustained release preparation containing Z11-hexadecenyl acetate was placed under the conditions of 40 °C, a wind speed of 0.5 m/sec. The release speed of Z11-hexadecenyl acetate from the preparation was measured by its weight change, giving a result showing good releasability that remaining ratio of Z11-hexadecenyl acetate was 15 % after 40 days.

### <Example 2-6>

60 parts by weight of ion-exchanged water was placed into a flask equipped with a stirrer, a thermometer, a cooler, a drip funnel and a nitrogen gas tube, and heated up to 65 °C under agitation and nitrogen gas flow. Then, 0.4 parts by weight of 2,2-azobis (2-amidinopropane) dihydrochloride is added as a polymerization initiator.

8 parts by weight of 7,8-epoxy-2-methyloctadecane (EMOD; sex pheromone of *Lymantria dispar Linnaeus* (diamondback moth)) as a functional substance, totally 21 parts by weight of monomer components (composition ratio is shown in Table 1), 1 part of polyoxyethylenealkyl ether sulfate ("PERSOFT" EL available from NOF CORPORATION; an anionic surface active agent) and 19 parts by weight of ion-exchanged water were placed into a beaker and stirred by a homo-mixer for 15 minutes at 10,000 rpm at ambient temperature to prepare a pre-emulsion.

Into the said flask, the pre-emulsion was dropped gradually taking 3 hours under the kept temperature condition of 65 °C, then further polymerized for 4 hours to obtain a emulsion (aqueous dispersion-type microgel A containing EMOD).

Totally 10 parts by weight of monomer components e and f previously mixed at composition ratio shown in Table 2 were dropped onto the obtained emulsion and radical polymerization reaction was proceeded under the irradiation of activated energy line to obtain an aqueous dispersion-type sustained release preparation containing EMOD in its IPN. Completion of the polymerization was confirmed in a same manner described in example 1.

The average particle size of the obtained microgel measured by dynamic light scattering method (DLS) was 130 nm.

To evaluate the aqueous dispersibility of the sustained release preparation, the emulsion was placed still in a temperature controlled bath at 25 °C and was studied deposition of coagulations. The result is shown in Table 3. The pH value for the emulsion is also shown in Table 3.

Further, to investigate its sustained releasability, the obtained aqueous dispersion-type sustained release preparation was dried to obtain a film of a sustained release preparation containing EMOD.

Next, these sustained release preparations containing EMOD were placed under the conditions of 35 °C and a wind speed of 1.5 m/sec, and the release speeds of EMOD from these preparations were measured by their weight changes. The results are shown in Fig. 1. As shown in Fig. 1, the release amounts of EMOD from these sustained release preparations had been kept almost constant during 40 days showing good sustained releasability.

### <Example 7>

Emulsion copolymerization and radical polymerization were performed in accordance with above-described examples using monomer components of the same composition ratio of example 3 and Z11-hexadecenyl acetate (sex pheromone of *Plutella xylostella Linnaeus* (diamondback moth)) instead of EMOD as a functional substance. Completion of the polymerization was confirmed in a same manner described in example 1. Obtained emulsion was not observed to deposit any coagulation etc. even after being kept still for a long time showing stabilized aqueous dispersibility. The pH value of the emulsion was 5.4.

Synthesized aqueous dispersion-type sustained release preparation containing Z11-hexadecenyl acetate was dried to obtain a film of a sustained release preparation containing Z11-hexadecenyl acetate.

Next, this sustained release preparation containing Z11-hexadecenyl acetate was placed under the conditions of 35 °C, a wind speed of 1.5 m/sec. The release speed of Z11-hexadecenyl acetate from the preparation was measured by its weight change, giving a result showing good releasability that remaining ratio of Z11-hexadecenyl acetate was 13 % after 40 days.

### <Example 8> (Reference Example)

60 parts by weight of ion-exchanged water was placed into a flask equipped with a stirrer, a thermometer, a cooler, a drip funnel and a nitrogen gas tube, and heated up to 65 °C under agitation and nitrogen gas flow. Then, 0.4 parts by weight of 2,2-azobis (2-amidino propane) dihydrochloride is added as a polymerization initiator.

8 parts by weight of 7,8-epoxy-2-methyloctadecane (EMOD; (sexpheromone of *Lymantria dispar Linnaeus* (gypsy moth)) as a functional substance, totally 20 parts by weight of monomer components (composition ratio is shown in Table 1), 1 part of polyoxyethylenealkyl ether sulfate ("PERSOFT" EL available from NOF CORPORATION; an anionic surface active agent) and 19 parts by weight of ion-exchanged water were placed into a beaker and stirred by a homo-mixer for 15 minutes at 10,000 rpm at ambient temperature to prepare a pre-emulsion.

Into the said flask, the pre-emulsion was dropped gradually taking 3 hours under the kept temperature condition of 65 °C, then further polymerized for 4 hours to obtain a emulsion (aqueous dispersion-type microgel A containing EMOD).

Totally 8 parts by weight of monomer components e and f previously mixed at composition ratio shown in Table 2 were dropped onto the obtained emulsion gradually taking 1 hour, then further stirred for 1 hour to impregnate monomers with microgel A. After that, 0.2 parts by weight of 2,2-azobis(2-amidinopropane) dihydrochloride were added and polymerized for 4 hours under the kept temperature condition of 65 °C to obtain an aqueous dispersion-type sustained release preparation containing EMOD in its IPN. Completion of the polymerization was confirmed in a same manner described in example 1.

The average particle size of the obtained microgel measured by dynamic light scattering method (DLS) was 230 nm.

To evaluate the aqueous dispersibility of the sustained release preparation, the emulsion was placed still in a temperature controlled bath at 25 °C and was studied deposition of coagulations. The result is shown in Table 3. The pH value for the emulsion is also shown in Table 3.

Further, to investigate its sustained releasability, the obtained aqueous dispersion-type sustained release preparation was dried to obtain a film of sustained release preparation containing EMOD.

Next, this sustained release preparation containing EMOD was placed under the conditions of 35 °C and a wind speed of 1.5 m/sec, and the release speed of EMOD from the preparation was measured by its weight change. The result is shown in Fig. 1. As shown in Fig. 1, this sustained release preparation showed relatively good sustained releasability although the release amount of EMOD had been slightly decreased from after 30 days until after 40 days.

### <Example 9>

60 parts by weight of ion-exchanged water was placed into a flask equipped with a stirrer, a thermometer, a cooler, a drip funnel and a nitrogen gas tube, and heated up to 65 °C under agitation and nitrogen gas flow. Then, 0.4 parts by weight of 2,2-azobis (2-amidino propane) dihydrochloride is added as a polymerization initiator.

8 parts by weight of 7,8-epoxy-2-methyloctadecane (EMOD; (sex pheromone of *Lymantria dispar Linnaeus* (gypsy moth)) as a functional substance, totally 20 parts by weight of monomer components (composition ratio is shown in Table 1), 1 part of polyoxyethylenealkyl ether sulfate ("PERSOFT" EL available from NOF CORPORATION; an anionic surface active agent) and 19 parts by weight of ion-exchanged water were placed into a beaker and stirred by a homo-mixer for 15 minutes at 10,000 rpm at ambient temperature to prepare a pre-emulsion.

Into the said flask, the pre-emulsion was dropped gradually taking 3 hours under the kept temperature condition of 65 °C, then further polymerized for 4 hours to obtain a emulsion (aqueous dispersion-type microgel A containing EMOD).

The obtained emulsion was transferred into an autoclave and totally 8 parts by weight of monomer components e and f previously mixed at composition ratio shown in Table 2 were dropped gradually taking 1 hour, then further stirred for 1 hour to impregnate monomers with microgel A. After that, polymerization was proceeded for 4 hours under the kept temperature condition of 120 °C to obtain an aqueous dispersion-type sustained release preparation containing EMOD in its IPN. Completion of the polymerization was confirmed in a same manner described in example 1.

The average particle size of the obtained microgel measured by dynamic light scattering method (DLS) was 210 nm.

To evaluate the aqueous dispersibility of the sustained release preparation, the emulsion was placed still in a temperature controlled bath at 25 °C and was studied deposition of coagulations. The result is shown in Table 3. The pH value for the emulsion is also shown in Table 3.

Further, to investigate its sustained releasability, the obtained aqueous dispersion-type sustained release preparation was dried to obtain a film of sustained release preparation containing EMOD.

Next, this sustained release preparation containing EMOD was placed under the conditions of 35 °C and a wind speed of 1.5 m/sec, and the release speed of EMOD from this preparation was measured by its weight change. The result is shown in Fig. 1. As shown in Fig. 1, the release amount of EMOD from this sustained release preparation had been kept almost constant during 40 days showing good sustained releasability.

### <Example 10>

Emulsion copolymerization and radical polymerization was performed in a same manner described in above examples using monomer components of same composition ratio of example 3, and using Z11-hexadecenal (sex pheromone of *Helicoverpa armigera Hubner*) instead of EMOD. Completion of the polymerization was confirmed in a same manner described in example 1. Obtained emulsion was not observed to deposit any coagulation etc. even after being kept still for a long time showing stabilized aqueous dispersibility. The pH value of the emulsion was 5.6.

Ion-exchanged water was added to the synthesized aqueous dispersion-type sustained release preparation containing Z11-hexadecenal to adjust to the resin content of 10 %. This sustained release preparation was applied uniformly onto a nylon fabric (size: 200 x 200mm) using a spray adjusting the adhered resin mass to approximately 5g/m2, and dried before its sustained releasability was evaluated.

Next, the sustained release preparation containing EMOD having been adhered to a nylon fabric was placed under the conditions of 25 °C and a wind speed of 2.0 m/sec, and the release speed of Z11-hexadecenal from this preparation was measured by GC analysis. The result is shown in Fig. 2. As shown in Fig. 2, the release amount of Z11-hexadecenal from this sustained release preparation had been kept almost constant during 40 days showing good sustained releasability.

### <Example 11>

The sustained releasability of the aqueous dispersion-type sustained release preparation containing EMOD obtained in example 3 was evaluated in a same manner described in example 10. The result is shown in Fig. 2. As shown in Fig. 2, the release amount of EMOD from this sustained release preparation had been kept almost constant during 40 days showing good sustained releasability.

### <Comparative Example 1>

Emulsion copolymerization was performed in a same manner described in above examples using monomer components a, c, d of same composition ratio in examples 3, 4, 6, and styrene was used instead of monomer component b (composition ratio is shown in Table 1). As the result, lots of coagulations were deposited while the first step polymerizations, and sustained release preparations having good aqueous dispersibility couldn't be obtained.

**Table 1**

| (parts by weight) | | | | | | | |
|---|---|---|---|---|---|---|---|
| component | a1 | a2 | b | | c | d | d |
| monomer | methacrylic ester having a photo-initiating group*1 | radical-polymerizing organic Peroxide*2 | lauryl methacrylate | styrene | neopentylglycol dimethacrylate | methoxy polyethyleneglycol monomethacrylate | methacrylic acid |
| Example 1 | 5 | - | 79 | - | 1 | - | 20 |
| Example 2 | 5 | - | 65 | - | 5 | 30 | - |
| Example 3 | 5 | - | 69 | - | 1 | 30 | - |
| Example 4 | 5 | - | 69 | - | 1 | 30 | - |
| Example 5 | 5 | - | 95 | - | 5 | - | - |
| Example 6 | 5 | - | 69 | - | 1 | 30 | - |
| Example 7 | 5 | - | 69 | - | 1 | 30 | - |
| Reference Example 8 | - | - | 69 | - | 1 | 30 | - |
| Example 9 | - | 5 | 69 | - | 1 | 30 | - |
| Example 10 | 5 | - | 69 | - | 1 | 30 | - |
| Example 11 | 5 | - | 69 | - | 1 | 30 | - |
| Comparative Example 1 | 5 | - | - | 69 | 1 | 30 | - |

In Table 1, component a1 is 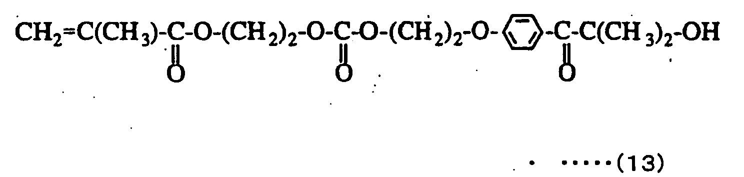 and component a2 is t-butylperoxymethacryloyloxyethyl carbonate.

**Table 2**

| (parts by weight) | | | |
|---|---|---|---|
| component | e | e | f |
| monomer | ethyl acrylate | butyl acrylate | neopentylarlycol neopentylglycol diacrylate |
| Example 1 | 90 | - | 10 |
| Example 2 | 90 | - | 10 |
| Example 3 | 90 | - | 10 |
| Example 4 | 95 | - | 5 |
| Example 5 | 90 | - | 10 |
| Example 6 | - | 90 | 10 |
| Example 7 | 90 | - | 10 |
| Reference Example 8 | 90 | - | 10 |
| Example 9 | 90 | - | 10 |
| Example 10 | 90 | - | 10 |
| Example 11 | 90 | - | 10 |
| Comparative Example 1 | - | - | - |

**Table 3**

| | aquaeous dispersion stability of sustained release preparation* | pH value of emulsion |
|---|---|---|
| Example 1 | excellent | 7 |
| Example 2 | excellent | 5.5 |
| Example 3 | excellent | 5.4 |
| Example 4 | excellent | 5.4 |
| Example 5 | good | 5.6 |
| Example 6 | excellent | 5.4 |
| Example 7 | excellent | 5.4 |
| Rererence Example 8 | good | 5.4 |
| Example 9 | good | 5.5 |
| Example 10 | excellent | 5.6 |
| Example 11 | excellent | 5.4 |
| Comparative Example 1 | poor | - |

| | | |
|---|---|---|
| * excellent: stable over 60 days. good: stable over 30 days. poor: coagulation generated after a few days. | | |

Aqueous dispersion-type sustained release preparations according to this invention can release almost all the functional substance contained inside of their interpenetrating polymer networks at a constant release speed for a considerable period of time, and easily applied by spraying or scattering, as well as is able to give more functions such as adhesivity etc. Thus, they are expected to be utilized in many fields.

## Claims

1. An aqueous dispersion-type sustained release preparation comprising:
a functional substance; and
interpenetrating polymer networks comprised of microgel A and microgel B,
wherein said functional substance is contained in said interpenetrating polymer networks, and said microgel A is a polymer formed by monomer components which comprise:
a radical-polymerizing organic peroxide (component a2) or a (meth)acrylic ester monomer having a photo-initiating group (component a1);
a (meth)acrylic ester monomer (component b);
a polyfunctional (meth)acrylic ester monomer (component c); and optionally, a hydrophilic monomer (component d), wherein said polymer has a good affinity to said functional substance whereas said microgel B is a polymer having poor affinity to said functional substance, and
wherein said microgel A and microgel B in the interpenetrating polymer networks are chemically bonded with each other through a fragment of (meth)acrylic ester monomer having a photo-initiating group (component a1) or a fragment of radical-polymerizing organic peroxide (component a2).

2. The aqueous dispersion-type sustained release preparation according to Claim 1, wherein said microgel B is formed by monomer components which comprise:
a (meth)acrylic ester monomer (component e);and
a polyfunctional (meth)acrylic ester monomer (component f).

3. The aqueous dispersion-type sustained release preparation according to any one of the preceding Claims, wherein said functional substance is a pheromone, and said (meth)acrylic ester monomer (component b) of said microgel A has an alkyl chain having 6-20 carbon atoms.

4. The aqueous dispersion-type sustained release preparation according to any one of the preceding Claims, wherein said functional substance is a pheromone, and said (meth)acrylic ester monomer (component e) of said microgel B has an alkyl chain having 1-8 carbon atoms.

5. The aqueous dispersion-type sustained release preparation according to any one of the preceding Claims, which is an emulsion having a pH value of 2-9.

6. A method for producing an aqueous dispersion-type sustained release preparation comprising a functional substance inside of interpenetrating polymer networks, said method comprising the steps of:
emulsion copolymerizing a plurality of monomer components which comprise:
a (meth)acrylic ester monomer having a photo-initiating group (component a1),
a (meth)acrylic ester monomer (component b),
a polyfunctional (meth)acrylic ester monomer (component c), and
optionally, a hydrophilic monomer (component d), together with a functional substance, a surface active agent, a polymerization initiator and water, under conditions such that the photo-initiating group in said component a1 does not decompose, to obtain an emulsion;
adding to the emulsion a (meth)acrylic ester monomer (component e) and a polyfunctional (meth)acrylic ester monomer (component f); and
polymerizing by irradiation of activated energy the emulsion, to obtain the aqueous dispersion-type sustained release preparation.

7. A method for producing an aqueous dispersion-type sustained release preparation comprising a functional substance inside of interpenetrating polymer networks, said method comprising the steps of:
emulsion copolymerizing a plurality of monomer components which comprise:
a radical-polymerizing organic peroxide (component a2),
a (meth)acrylic ester monomer (component b),
a polyfunctional (meth)acrylic ester monomer (component c), and
optionally, a hydrophilic monomer (component d), together with a functional substance, a surface active agent, a polymerization initiator and water, under conditions such that the peroxy bond in said component a2 does not decompose, to obtain a emulsion;
adding to the emulsion a (meth)acrylic ester monomer (component e) and a polyfunctional (meth)acrylic ester monomer (component f) and
polymerizing the emulsion under conditions such that the peroxy bond in said component a2 decomposes, to obtain the aqueous dispersion-type sustained release preparation.

## Patentansprüche

1. Formulierung vom wässrigen Dispersionstyp mit verzögerter Freigabe, umfassend:
eine funktionelle Substanz, und
sich durchdringende Polymernetzwerke, umfassend Mikrogel A und Mikrogel B,
wobei die funktionelle Substanz in den sich durchdringenden Polymernetzwerken enthalten ist, und das Mikrogel A ein Polymer ist, welches durch MonomerKomponenten gebildet ist, die umfassen:
ein radikalisch polymerisierendes organisches Peroxid (Komponente a2) oder ein (Meth)acrylestermonomer mit einer photo-initiierenden Gruppe (Komponente a1),
ein (Meth)acrylestermonomer (Komponente b),
ein polyfunktionelles (Meth)acrylestermonomer (Komponente c), und
gegebenenfalls ein hydrophiles Monomer (Komponente d),
wobei das Polymer eine gute Affinität zu der funktionellen Substanz aufweist, wohingegen das Mikrogel B ein Polymer mit einer geringen Affinität zu der funktionellen Substanz ist, und
wobei das Mikrogel A und das Mikrogel B in den sich durchdringenden Polymernetzwerken durch ein Fragment von (Meth)acrylestermonomer mit einer photo-initiierenden Gruppe (Komponente a1) oder ein Fragment von radikalisch polymerisierendem organischem Peroxid (Komponente a2) chemisch miteinander verbunden sind.

2. Formulierung vom wässrigen Dispersionstyp mit verzögerter Freigabe nach Anspruch 1, wobei das Mikrogel B durch Komponenten gebildet ist, die ein (Meth)acrylestermonomer (Komponente e), und
ein polyfunktionelles (Meth)acrylestermonomer (Komponente f) umfassen.

3. Formulierung vom wässrigen Dispersionstyp mit verzögerter Freigabe nach einem der vorhergehenden Ansprüche, wobei die funktionelle Substanz ein Pheromon ist und das (Meth)acrylestermonomer (Komponente b) des Mikrogels A eine Alkylkette mit 6 bis 20 Kohlenstoffatomen aufweist.

4. Formulierung vom wässrigen Dispersionstyp mit verzögerter Freigabe nach einem der vorhergehenden Ansprüche, wobei die funktionelle Substanz ein Pheromon ist und das (Meth)acrylestermonomer (Komponente e) des Mikrogels B eine Alkylkette mit 1 bis 8 Kohlenstoffatomen aufweist.

5. Formulierung vom wässrigen Dispersionstyp mit verzögerter Freigabe nach einem der vorhergehenden Ansprüche, welche eine Emulsion mit einem pH-Wert von 2 bis 9 ist.

6. Verfahren zur Herstellung einer Formulierung vom wässrigen Dispersionstyp mit verzögerter Freigabe, welche eine funktionelle Substanz in den sich durchdringenden Polymernetzwerken umfasst, wobei das Verfahren die Schritte
des Emulsionscopolymerisierens einer Vielzahl von Monomer-Komponenten, welche
ein (Meth)acrylestermonomer mit einer photo-initiierenden Gruppe (Komponente a1),
ein (Meth)acrylestermonomer (Komponente b),
ein polyfunktionelles (Meth)acrylestermonomer (Komponente c), und
gegebenenfalls ein hydrophiles Monomer (Komponente d), umfassen,
zusammen mit einer funktionellen Substanz, einem grenzflächenaktiven Mittel, einem Polymerisationsinitiator und Wasser, unter Bedingungen, unter welchen sich die photo-initiierende Gruppe in der Komponente a1 nicht zersetzt, um eine Emulsion zu erhalten,
des Hinzufügens eines (Meth)acrylestermonomers (Komponente e) und eines polyfunktionellen (Meth)acrylestermonomers (Komponente f) zu der Emulsion, und
des Polymerisierens der Emulsion durch Bestrahlung mit aktivierter Energie, um die Formulierung vom wässrigen Dispersionstyp mit verzögerter Freigabe zu erhalten,
umfasst.

7. Verfahren zur Herstellung einer Formulierung vom wässrigen Dispersionstyp mit verzögerter Freigabe, welche eine funktionelle Substanz in den sich durchdringenden Polymernetzwerken umfasst, wobei das Verfahren die Schritte
des Emulsionscopolymerisierens einer Vielzahl von Monomer-Komponenten, welche
ein radikalisch polymerisierendes organisches Peroxid (Komponente a2),
ein (Meth)acrylestermonomer (Komponente b),
ein polyfunktionelles (Meth)acrylestermonomer (Komponente c), und gegebenenfalls ein hydrophiles Monomer (Komponente d), umfassen,
zusammen mit einer funktionellen Substanz, einem grenzflächenaktiven Mittel, einem Polymerisationsinitiator und Wasser, unter Bedingungen, unter welchen sich die Peroxy-Bindung in der Komponente a2 nicht zersetzt, um eine Emulsion zu erhalten,
des Hinzufügens eines (Meth)acrylestermonomers (Komponente e) und eines polyfunktionellen (Meth)acrylestermonomers (Komponente f) zu der Emulsion, und
des Polymerisierens der Emulsion unter Bedingungen, unter welchen sich die Peroxy-Bindung in der Komponente a2 zersetzt, um die Formulierung vom wässrigen Dispersionstyp mit verzögerter Freigabe zu erhalten,
umfasst.

## Revendications

1. Préparation de type dispersion aqueuse à libération prolongée comprenant :
une substance fonctionnelle ; et
des réseaux polymères d'interpénétration constitués de microgel A et de microgel B,
ladite substance fonctionnelle étant contenue dans lesdits réseaux polymères d'interpénétration et ledit microgel A étant un polymère formé par des composants monomères qui comprennent :
un peroxyde organique à polymérisation radicalaire (composant a2) ou un monomère d'ester (méth)acrylique ayant un groupe photo-initiateur (composant a1) ;
un monomère d'ester (méth)acrylique (composant b) ;
un monomère d'ester (méth)acrylique polyfonctionnel (composant c) ; et éventuellement, un monomère hydrophile (composant d), ledit polymère ayant une bonne affinité avec ladite substance fonctionnelle alors que ledit microgel B est un polymère ayant une faible affinité avec ladite substance fonctionnelle, et
ledit microgel A et ledit microgel B dans les réseaux polymères d'interpénétration étant liés chimiquement l'un à l'autre par un fragment de monomère d'ester (méth)acrylique ayant un groupe photo-initiateur (composant a1) ou un fragment de peroxyde organique à polymérisation radicalaire (composant a2).

2. Préparation de type dispersion aqueuse à libération prolongée selon la revendication 1, dans laquelle ledit microgel B est formé par des composants monomères qui comprennent :
un monomère d'ester (méth)acrylique (composant e) ; et
un monomère d'ester (méth)acrylique polyfonctionnel (composant f).

3. Préparation de type dispersion aqueuse à libération prolongée selon l'une quelconque des revendications précédentes, dans laquelle ladite substance fonctionnelle est une phéromone et ledit monomère d'ester (méth)acrylique (composant b) dudit microgel A a une chaîne alkyle ayant 6 à 20 atomes de carbone.

4. Préparation de type dispersion aqueuse à libération prolongée selon l'une quelconque des revendications précédentes, dans laquelle ladite substance fonctionnelle est une phéromone et ledit monomère d'ester (méth)acrylique (composant e) dudit microgel B a une chaîne alkyle ayant 1 à 8 atomes de carbone.

5. Préparation de type dispersion aqueuse à libération prolongée selon l'une quelconque des revendications précédentes, qui est une émulsion ayant un pH de 2 à 9.

6. Procédé de production d'une préparation de type dispersion aqueuse à libération prolongée comprenant une substance fonctionnelle à l'intérieur des réseaux polymères d'interpénétration, ledit procédé comprenant les étapes suivantes :
copolymériser en émulsion une pluralité de composants monomères comprenant :
un monomère d'ester (méth)acrylique ayant un groupe photo-initiateur (composant a1),
un monomère d'ester (méth)acrylique (composant b),
un monomère d'ester (méth)acrylique polyfonctionnel (composant c), et
éventuellement, un monomère hydrophile (composant d), conjointement avec une substance fonctionnelle, un agent tensioactif, un initiateur de polymérisation et de l'eau, dans des conditions telles que le groupe photo-initiateur dans ledit composant a1 ne se décompose pas, pour obtenir une émulsion ;
ajouter à l'émulsion un monomère d'ester (méth)acrylique (composant e) et un monomère d'ester (méth)acrylique polyfonctionnel (composant f) ; et
polymériser l'émulsion par irradiation d'énergie activée, pour obtenir la préparation de type dispersion aqueuse à libération prolongée.

7. Procédé de production d'une préparation de type dispersion aqueuse à libération prolongée comprenant une substance fonctionnelle à l'intérieur de réseaux polymères d'interpénétration, ledit procédé comprenant les étapes suivantes :
copolymériser en émulsion une pluralité de composants monomères comprenant :
un peroxyde organique de polymérisation radicalaire (composant a2),
un monomère d'ester (méth)acrylique (composant b),
un monomère d'ester (méth)acrylique polyfonctionnel (composant c), et
éventuellement, un monomère hydrophile (composant d), conjointement avec une substance fonctionnelle, un agent tensioactif, un initiateur de polymérisation et de l'eau, dans des conditions telles que la liaison peroxy dans ledit composant a2 ne se décompose pas, pour obtenir une émulsion ;
ajouter à l'émulsion un monomère d'ester (méth)acrylique (composant e) et un monomère d'ester (méth)acrylique polyfonctionnel (composant f) ; et
polymériser l'émulsion dans des conditions telles que la liaison peroxy dans ledit composant a2 se décompose, pour obtenir la préparation de type dispersion aqueuse à libération prolongée.
